# EUROPEAN PATENT APPLICATION

(11) **EP 4 810 620 A1**
(43) Date of publication of application: **23.09.2026**
(21) Application number: 25216889.3
(22) Date of filing: 19.11.2025
(51) Int. Cl.: C12P 7/10, C08B 1/00, C08B 37/00, C08H 8/00, C12P 19/02, C12P 19/14, D21C 3/06

(54) **PROCESS FOR THE PRODUCTION OF FERMENTATION PRODUCTS AND LIGNIN**

(30) Priority: 18.03.2025 US 202563773846 P; 11.11.2025 US 202519385649
(71) Applicant: American Process International LLC, Atlanta, Georgia 30309 (US)
(72) Inventor: IAKOVLEV, Mikhail, Atlanta, GA 30309 (US); RETSINA, Theodora, Atlanta, GA 30309 (US); RUTHERFORD, Steve, Atlanta, GA 30309 (US); HILL, Lee, Atlanta, GA 30309 (US); MAINA, Sofia, 15771 Zografou (GR); EFTHYMIOU, Maria-Nefeli, 18755 Keratsini (GR); DEZE, Evangelia G., 32200 Thiva (GR); PAPAIOANNOU, Myrto, 15561 Cholargos (GR)
(74) Representative: FRKelly

(57) **Abstract**

A process for the production of one or more fermentation products and lignin. The method comprises pretreatment with sulfur dioxide and a volatile alcohol, chemical recovery, whole slurry saccharification, and fermentation. High monosaccharide yields are achieved, and alkoxylated and/or non-alkoxylated lignin fractions, as well as lignosulfonate, can be produced and separated if desired. Monosaccharides with or without lignin are subjected to fermentation to produce one or more fermentation products, for example, ethanol.

## Description

### RELATED APPLICATIONS

This application claims priority to U.S. Application No. 19/385,649 filed November 11, 2025 and U.S. Provisional Patent Application No. 63/773,846 filed March 18, 2025, which applications are incorporated herein by reference.

### BACKGROUND OF THE INVENTION

Economically efficient conversion of lignocellulosic biomass to monosaccharides, lignin and fermentation products is challenging due to the recalcitrant nature of lignocellulosic biomass. The key step of such conversion is a pretreatment (or fractionation) process. The typical problems of the known pretreatment/fractionation processes are high sugar degradation and high fermentation inhibitor formation, low enzymatic digestibility of cellulose, low yields of monomeric sugars and fermentation products, low yield of lignin, low quality lignin (condensed, derivatized, etc.), sticky lignin precipitates plugging equipment, expensive/toxic chemicals, low chemical recovery rates, and the like.

Known pretreatment processes include, among others, hot water, steam explosion, dilute acid, NREL's deacetylation-mechanical refining (DMR), sulfite/SPORL, sulfur dioxide, organosolv (organic solvent-water with or without acid/alkali catalyst), and SO₂-organic solvent-water processes (for example, the OR1 Process described in US Patent No. 11,118,017 (lakovlev et al.), and the AVAP^{®} process).

**Hot water (hydrothermal), steam explosion and dilute acid pretreatment processes** (150 °C - 220 °C) are characterized by high sugar degradation, lignin condensation, low enzymatic digestibility of cellulose, formation of sticky lignin precipitates, non-applicability to softwoods, and in case of dilute acid, acid recovery challenges. For example, Perez et al. (2008) reported at least 47% hemicellulose sugar loss in optimized hot water pretreatment of wheat straw. In another report, dilute sulfuric acid pretreatment of pine resulted in only 43% glucan-to-glucose conversion at enzyme dosage of about 22 filter-paper units (FPU)/g glucan. Total monomeric sugar recovery was only 248 kg per 'bone dry' (BD) tonne biomass feed (Zhu et al., 2010).

**NREL's Deacetylation-mechanical refining (DMR) process** (Chen et al. 2024, Chen et al. 2016) utilizes deacetylation of hemicelluloses by alkali solution followed by mechanical refining. The treatment leads to rather low sugar yields at high enzyme dosages, presumably due to the limited effect on the cell wall structure whereby most of the lignin and hemicelluloses remain with cellulose preventing the enzyme accessibility. The total monosaccharide yields based on carbohydrates in raw material reported for corn stover are about 80% using 16 mg CTec3 protein/g cellulose (12 FPU/g cellulose) and 4 mg HTec3 protein/g cellulose and about 74% using 8 mg CTec3 protein/g cellulose (6 FPU/g cellulose) and 2 mg HTec3 protein/g cellulose (Chen et al. 2016). Furthermore, the alkali regeneration cannot be easily accomplished, adding to the costs of the process.

**Sulfite pulping and SPORL pretreatment processes** (EP 2376642 (Sjoede) "LIGNOCELLULOSIC BIOMASS CONVERSION BY SULFITE PRETREATMENT", Tian et al. 2011, Wang et al. 2012) utilize sulfur dioxide and salts of sulfurous acids, and in case of SPORL also occasionally sulfuric acid. Temperatures between 125 and 180 °C are used, and pH ranges from 1 to 13, mostly in acidic range. The processes reportedly perform better than dilute acid pretreatment in terms of cellulose enzymatic digestibility (Tian et al. 2011, Wang et al. 2012). However, the presence of hydrogen sulfite anions and acidity leads to sugar degradation. Large portion of monosaccharides (>20-30%, Pfister and Sjöström 1977) is lost as aldonic acids. The total monomeric sugar recovery was reported at about 520 kg per BD tonne of Douglas Fir (Zhu et al., 2015). Recovery of a complex mixture of hydrogen sulfites, sulfites, thiosulfates, and sulfates presents a hardly solvable challenge.

**Sulfur dioxide-catalyzed pretreatment processes** (Soderstrom et al. 2004, Stenberg et al. 1998, Galbe and Zacchi 2002, Ewanick et al. 2007, and van der Meulen: EP 2516660, US 8,834,633 (Van Der Meulen et al.), and US 9,528,129 (Van Der Meulen et al.)) generally utilize the SO₂ concentration in the liquid at below 3%, temperatures of about 150-215 °C, and the pH of the liquors after pretreatment is generally 2-3, indicating that the residual concentration of sulfur dioxide is low. Low SO₂ pretreatments behave similarly to dilute acid pretreatments as most SO₂ is consumed to form strong lignosulfonic acid, and thus lignin condensation and reprecipitation on fibers are observed and sticky precipitates are expected. A high residual lignin content of the cellulosic fibers results in lower than optimal enzymatic digestibility of cellulose, with only 60-70% glucan-to-glucose conversion at enzyme dosages of higher or substantially higher than 20 FPU/g glucan. Total monomeric sugar yields of only about 470-520 kg per BD tonne biomass feed or below have been reported (Soderstrom et al., 2004, Stenberg et al., 1998, Galbe and Zacchi, 2002, Ewanick et al., 2007). High SO₂ pretreatment results in considerably higher sugar yield and efficient lignin solubilization, for example, as described in US Patent No. 11,306,113 (lakovlev et al.). However, these processes convert most of the lignin to a derivative, i.e., lignosulfonic acid, having a lower application potential compared to the non-sulfonated (native) form of lignin.

In **organosolv processes** (see US Patent No. 1,856,567 (Kleinert and Tayenthal), issued on May 3, 1932), lignocellulosic biomass is treated with an organic solvent and water in various ratios with or without the presence of catalyst, typically at temperatures of 160-210 °C. The advantage of organosolv processes is that the presence of an organic solvent promotes dissolution of lignin, which is said to be in near-native form, although the lignin becomes alkoxylated to an extent when alcohol is used as solvent. The most common organic solvents are alcohols, ketones, esters, organic acids, while the most common catalyst is sulfuric acid. The combination of high temperature and acidity in the organosolv process results in high sugar degradation, especially in uncatalyzed organosolv processes, where approximately 30% sugar loss is observed (van Heiningen et al. 2018).

Another disadvantage of organosolv processes is the limited delignification and limited removal of hemicelluloses from cellulose. Uncatalyzed organosolv processing of softwoods results in a high residual lignin content of the fibers. For example, Aziz and Sarkanen 1989, report that uncatalyzed softwood organosolv pulps have a high residual lignin content (kappa number 80-100, i.e., about 13-20% residual lignin). After uncatalyzed organosolv pulping at 185 °C for 60 minutes, Kleinert (1974) obtained cellulosic pulp from spruce with about 5% residual lignin and 8% residual hemicelluloses at liquor-to-solid ratio of 10, which shows inefficient removal of hemicelluloses from softwoods, even at the very high liquor-to-solid ratio. At lower liquor-to-solid ratio, the delignification is considerably impaired.

The presence of catalysts increases the organosolv pretreatment efficiency, but it is still a challenge to produce pure cellulosic pulps. For example, mixed softwoods consisting of spruce, pine, and Douglas fir from lumber mill whitewood residues pretreated in 60% ethanol with sulfuric acid as a catalyst (185-198 °C for 30-60 minutes, pH 2.0-3.4; liquor-to-solid ratio 7-10 kg/kg) produced cellulosic pulps with residual lignin content varying from 6.4 to 27.4% (Pan et al. 2005). Residual organosolv lignin, especially in non-sulfonated form, is known to decrease enzymatic digestibility by increasing non-productive enzyme adsorption on lignin (Nakagame et al. 2010, del Rio et al. 2011).

For uncatalyzed organosolv processes where the solvents are volatile alcohols, ketones, esters, or other volatile compounds, the solvent in the spent pulping liquor can readily be recovered by distillation. However, alcohol chemically bound to lignin and sugars in organosolv processes is difficult to recover without addition of an acidic catalyst, which likely explains the high ethanol losses in the ethanol-based Alcell process.

In organosolv processes with a volatile solvent, the solvent needs to be recovered, for example, by distillation, which results in lignin precipitation, because organosolv lignin is not soluble in water. The addition of organosolv lignin to cellulose decreases the enzyme hydrolysis yields. Thus, in order to avoid lignin precipitation on cellulose, a cellulose washing step, i.e., the separation of cellulose and dissolved material, is used prior to the solvent recovery step. No literature reference to an organosolv pretreatment process without cellulose washing, and with the whole slurry undergoing solvent recovery followed by enzyme hydrolysis, has been found.

**SO₂-alcohol-water processes** (OR1 Process, US Patent No. 11,118,017 (lakovlev et al.), and the AVAP^{®} process: US Patent Nos. 8,038,842 and 8,268,125, both to Retsina et al.) utilize sulfur dioxide, alcohol and water at temperatures of about 135-165 °C. The AVAP^{®} process utilizes sulfur dioxide concentrations of over 9%, while OR1 Process utilizes sulfur dioxide concentrations of about 3-8%. Both allow for efficient delignification and high sugars yields, however, the AVAP^{®} process favors lignin sulfonation to produce lignosulfonic acid, which results in lower lignin yields compared to the OR1 Process. Similar to the organosolv processes, existing SO₂-alcohol-water processes require large amounts of unit operations, including separation of cellulosic pulp and dissolved material and washing, enzyme hydrolysis of cellulosic pulp, sulfur dioxide and ethanol recovery, and heat treatment of dissolved material to increase sugar yield.

Recovery of chemicals is a decisive factor for the economic viability of a pretreatment process. Non-volatile inorganic acids (dilute acid, SPORL), alkalis, and salts (sulfite, SPORL) cannot be efficiently recovered, while sulfur dioxide and volatile organic solvent are relatively easy to recover, for example, by flashing and/or steam stripping. Difficulty recovering the base is a major drawback of most base-utilizing processes.

Enzymatic digestibility of cellulose is a key factor for an economic process. It is common knowledge that enzymatic digestibility of cellulose is most efficient when the cellulose purity is high, i.e., the cellulose contains only small amounts of residual hemicelluloses and lignin. Lignin in solid, colloidal or dissolved forms is known to cause non-productive enzyme adsorption resulting in low glucose yields. Lignin is known to be inhibitory to enzymatic hydrolysis (Lai et al. 2014, Pielhop et al. 2015, Huang et al. 2017). Thus, processes producing such lignin fractions (hot water, steam explosion, dilute acid, organosolv, SO₂-alcohol-water, etc.) require separation of cellulosic pulp from liquor (termed pulp washing) in order to avoid the inhibitory effect of the lignin fractions on enzymatic digestibility. This increases the capital and operation costs of a Biorefinery.

It is also conventional knowledge that fiber explosion (e.g., rapid decrease in pressure leading to instantaneous evaporation of liquid within the fiber structure) after pretreatment results in increased enzymatic digestibility of cellulose, presumably due to disruption of the fiber cell wall structure providing improved enzyme accessibility to the cellulose surface, after the explosive decompression.

Other than the OR1 process, which involves the separation of cellulose pulp and dissolved material, current pretreatment processes do not achieve ***simultaneous*** high-yield production of fermentable monomeric sugars and non-condensed lignin, in an economical manner without producing sticky lignin precipitates. High yield of fermentable monomeric sugars is defined for softwoods (gymnosperms) as higher than about 75% based on available polysaccharides in the biomass at an enzyme charge of about 3.3 FPU/o.d. g biomass or lower ("o.d." refers to oven dried biomass basis). High yield of fermentable monomeric sugars is defined for hardwoods and herbaceous (non-woody) lignocellulosic biomass (angiosperms) as higher than about 80% based on available polysaccharides in the biomass at an enzyme charge of about 1.6 FPU/o.d. g biomass or lower. The total sugar-derived inhibitors (i.e., furfural, HMF, and levulinic acid) should be low, i.e., less than about 10 kg per BD metric tonne biomass. High lignin yields are defined as higher than 65% based on lignin in biomass (or, for softwoods, higher than 180 BD kg lignin per BD metric tonne biomass). Furthermore, acidic pretreatment processes, for example, those utilizing sulfuric acid as catalyst, often suffer from creation of lignin sticky precipitates that clog the processing equipment resulting in increased downtime. Accordingly, there is a need for improved processes for converting lignocellulosic biomass to non-condensed lignin fractions, lignocellulosic sugars, and fermentation products, in high yields, without the production of lignin sticky precipitates that clog the processing equipment, wherein the processes do not require separation of cellulose pulp and dissolved material and the processes can hydrolyze and ferment the 'whole slurry' after pretreatment, which collectively result in much lower capital and operating costs.

### SUMMARY

The invention provides a process for lignocellulosic biomass conversion to monosaccharides, lignin fractions and one or more fermentation products, resulting in over 76-83% of available saccharides converted to monosaccharides for softwoods (such as pine, spruce, Douglas fir, larch, cedar, etc.) and over 81-93% of available saccharides converted to monosaccharides for hardwoods (such as birch, beech, eucalyptus, aspen, poplar, maple, oak, etc.) and for herbaceous biomass/agricultural residues/energy crops (such as corn stover, corn cobs, wheat straw, barley straw, rice straw, rice husk, sugarcane straw, energy cane, sorghum stover, miscanthus, switchgrass, giant reed, elephant grass, kenaf, hemp residues, empty fruit bunches, etc.), lignin converted to non-condensed reactive lignin (in alkoxylated and/or non-alkoxylated forms) and optionally lignosulfonate. The process involves pretreatment with a solution of volatile alcohol containing sulfur dioxide, followed by recovery of chemicals, cellulose saccharification, and fermentation of sugars. Non-condensed lignin and lignosulfonate fractions can be separated after saccharification, after fermentation, or after separation of the fermentation product(s). The innovative combination of process steps combined as described herein provides significantly increased yields of both sugars and non-condensed lignin from lignocellulosic biomass and significant simplification of downstream processing. Compared to existing processes utilizing organic solvents (organosolv, SO₂-alcohol-water, and other processes), the step of pulp washing is eliminated. As a result of these improvements, a highly efficient and economical biorefinery process has been achieved.

One aspect of the invention comprises a process for the production of one or more fermentation products and lignin, comprising (a) contacting lignocellulosic biomass or steamed lignocellulosic biomass with a pretreatment liquor at an elevated temperature (such as a temperature of about 120 °C to about 190 °C) in a closed system under pressure; thereby releasing and/or dissolving lignin and hemicelluloses from the lignocellulosic biomass into the pretreatment liquor, resulting in a mixture comprising lignin dissolved in the pretreatment liquor, hemicelluloses dissolved and hydrolyzed to monosaccharides in the pretreatment liquor, and a solid fraction comprising mainly cellulose from the lignocellulosic biomass, which mixture is referred to as the pretreated material.

The process further includes one or more of (b) recovering sulfur dioxide and alcohol, thereby precipitating lignin, producing a first mixture comprising cellulose, lignin, and monosaccharides; (c) adjusting the pH of the first mixture (or a modified first mixture) to an appropriate level for enzymatic hydrolysis; (d) contacting the pH-adjusted first mixture with a cellulase, a glucosidase, a hemicellulase, a glycosidase or a combination thereof, to provide a subsequent mixture comprising glucose derived from cellulose and monosaccharides derived from hemicelluloses as well as lignin (alkoxylated lignin and/or non-alkoxylated lignin); and (e) subjecting the subsequent mixture to fermentation.

### BRIEF DESCRIPTION OF THE DRAWINGS

The following drawings form part of the specification and are included to further demonstrate certain embodiments or various aspects of the invention. In some instances, embodiments of the invention can be best understood by referring to the accompanying drawings in combination with the detailed description presented herein. The description and accompanying drawings may highlight a certain specific example, or a certain aspect of the invention. However, one skilled in the art will understand that portions of the example or aspect may be used in combination with other examples or aspects of the invention described herein.

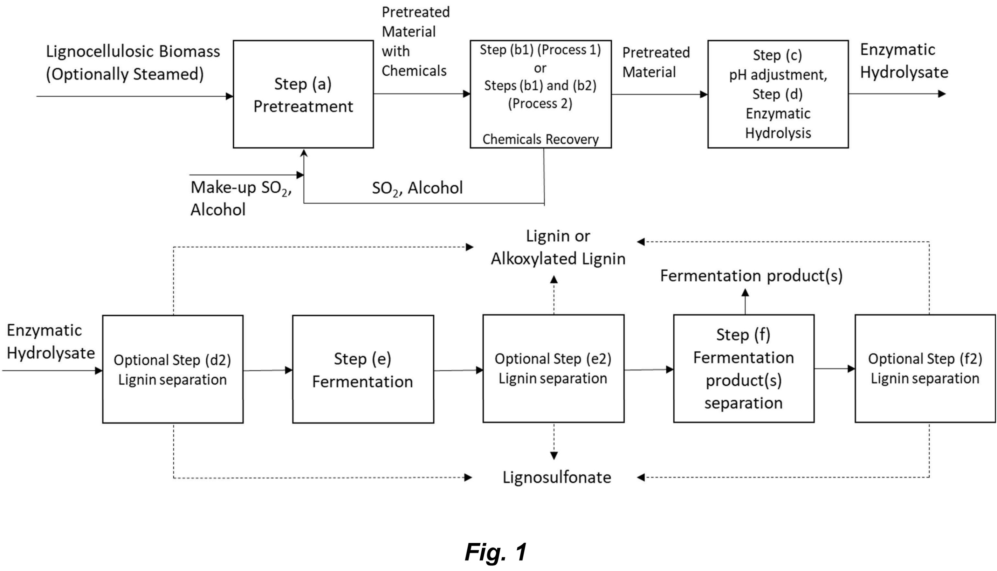

**Figure 1****.** A flowsheet example of the process for the production of one or more fermentation products and lignin fractions. The lignin fractions can be isolated at various points in the process to provide commercial products, in addition to the production of the one or more fermentation products.

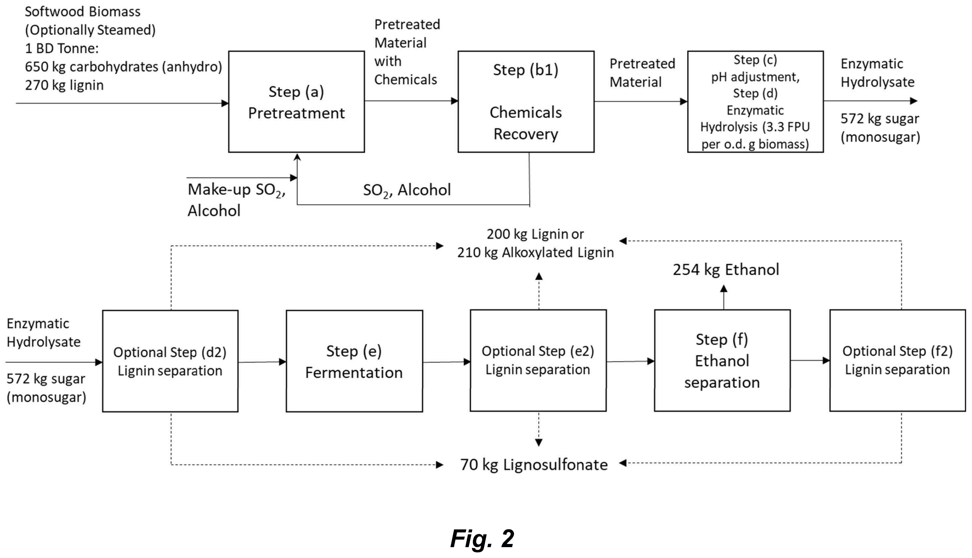

**Figure 2****.** Example of a Process 1 flow diagram wherein the biomass feed is softwood (e.g., pine or spruce) and the fermentation product is ethanol, with product yields based on 1 metric tonne of dry biomass. Typical softwood biomass containing 650 kg polysaccharides per 1 metric tonne of dry biomass was used. The figure corresponds to Example 1.1 (monosaccharide yield is 79% on available polysaccharides in biomass feedstock). The fermentation yield of 87% (% of theoretical) is based on the yields obtained from separate fermentations of a similar composition. The lignin and lignosulfonate yields were determined in a separate experiment.

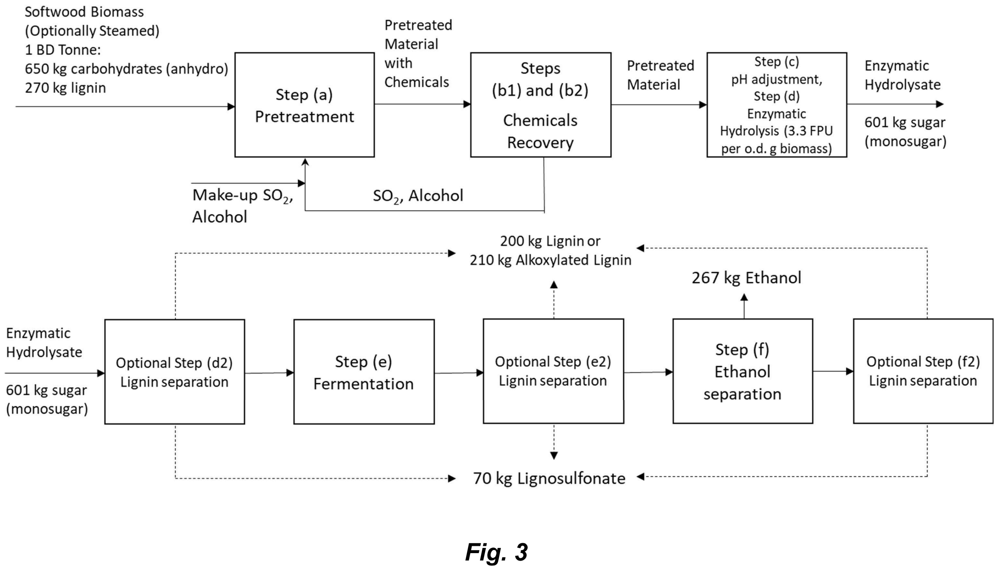

**Figure 3****.** Example of a Process 2 flow diagram wherein the biomass feed is softwood (e.g., pine or spruce) and the fermentation product is ethanol, with product yields based on 1 metric tonne of dry biomass. Typical softwood biomass containing 650 kg polysaccharides per 1 metric tonne of dry biomass was used. The figure corresponds to Example 1.7 (monosaccharide yield is 83% on available polysaccharides in biomass feedstock). The fermentation yield of 87% (% of theoretical) is based on the yields obtained from separate fermentations of a similar composition. The lignin and lignosulfonate yields were determined in a separate experiment.

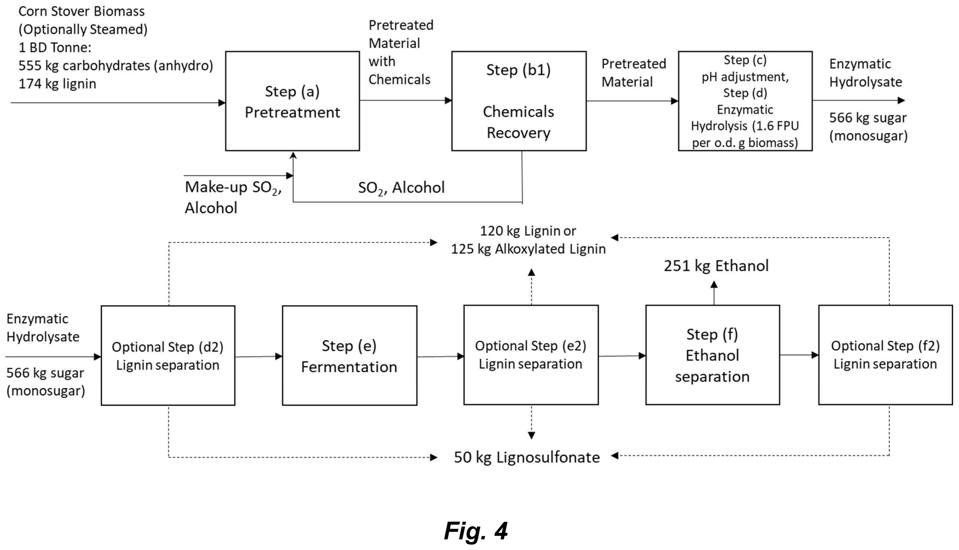

**Figure 4****.** Example of Process 1 flow diagram wherein the biomass feed is corn stover (agricultural residue) and the fermentation product is ethanol, with product yields based on 1 metric tonne of dry biomass. Corn stover used in the experiments contained 555 kg polysaccharides per 1 metric tonne of dry biomass. The figure corresponds to Example 2.9 (monosaccharide yield of 91% is based on available polysaccharides in biomass feedstock). The fermentation yield of 87% (% of theoretical) is based on the results obtained in Example 4. The lignin and lignosulfonate yields are yields calculated from separate pine results.

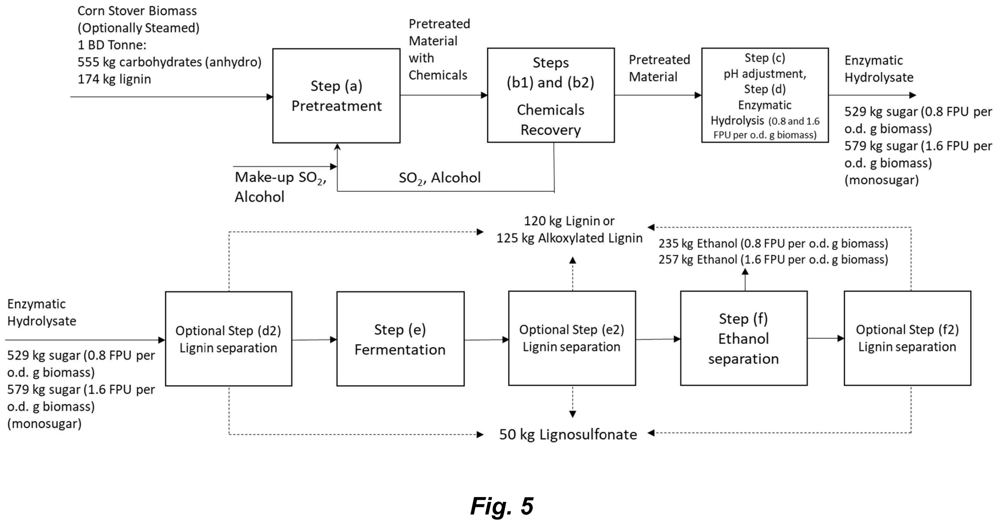

**Figure 5****.** Example of Process 2 flow diagram wherein the biomass feed is corn stover (agricultural residue) and the fermentation product is ethanol, with product yields based on 1 metric tonne of dry biomass. Corn stover used in the experiments contained 555 kg polysaccharides per 1 metric tonne of dry biomass. The figure corresponds to Examples 2.3 and 2.4 (monosaccharide yields are 85 and 93% on available polysaccharides in biomass feedstock). The fermentation yield of 87% (% theoretical) is based on the results obtained in Example 4. The lignin and lignosulfonate yields are yields calculated from separate pine results.

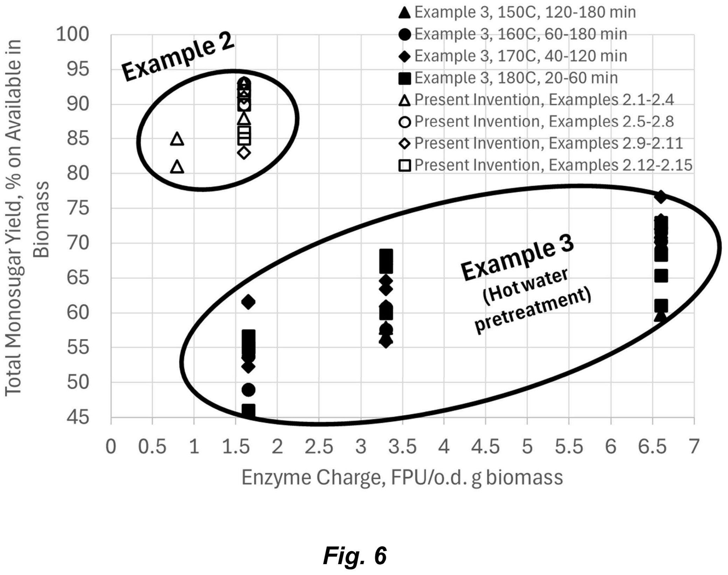

**Figure 6****.** The monosugar yields from corn stover biomass obtained by the method described in Example 2 in comparison with those obtained in a conventional hot water (hydrothermal) pretreatment (Example 3).

### DETAILED DESCRIPTION

The present invention generally relates to pretreatment processes for converting lignocellulosic biomass to various products including but not limited to fermentable sugars and lignin fractions, wherein the pretreatment process is carried out in a closed system. As used herein, a lignin fraction refers to one or more of lignin, alkoxylated lignin, and lignosulfonate. The sugars are fermented to one or more of the fermentation products. Fermentation products can include, for example, ethanol, other alcohols, organic acids, biopolymers, microbial oil, microbial protein, yeast biomass, and the like.

### Process 1.

In a first embodiment, referred to herein as Process 1, this disclosure provides a process for the production of one or more fermentation products and lignin from lignocellulosic biomass comprising:
(a) contacting lignocellulosic biomass or steamed lignocellulosic biomass with a pretreatment liquor at an elevated temperature under pressure;
   wherein the pretreatment liquor comprises sulfur dioxide, a volatile alcohol, and water;
   thereby releasing and/or dissolving lignin and hemicelluloses from the lignocellulosic biomass into the pretreatment liquor, resulting in a mixture comprising lignin and hemicelluloses dissolved in the pretreatment liquor and a solid fraction comprising mainly cellulose from the lignocellulosic biomass, which mixture is referred to as the pretreated material;
(b1) recovering sulfur dioxide and alcohol, thereby precipitating lignin, producing a first mixture comprising cellulose, lignin, and monosaccharides;
(c) adjusting the pH of the first mixture to an appropriate level for enzymatic hydrolysis, for example, 4 to 7, 4.5 to 6.5, or 5 to 6;
(d) contacting the pH-adjusted first mixture with a cellulase, a glucosidase, a hemicellulase, a glycosidase or a combination thereof, to provide a second mixture comprising glucose derived from cellulose and monosaccharides derived from hemicelluloses as well as lignin;
(d2) optionally removing lignin, alkoxylated lignin and/or lignosulfonate from the second mixture providing third mixture;
(e) subjecting the second or third mixture to fermentation, optionally in combination with adjusting the pH of the second or third mixture, for example, to 3.5 to 7, to 4.5 to 6.5, or to about 5 to about 6, to produce fermented broth containing one or more fermentation products;
(e2) optionally removing lignin, alkoxylated lignin and/or lignosulfonate from the fermented broth;
(f) separating the one or more fermentation products, optionally using a beer column, and providing fermentation product-lean mixture (for example, stillage); and
(f2) optionally removing lignin, alkoxylated lignin and/or lignosulfonate from the fermentation product-lean mixture.

In some embodiments, the elevated temperature of step (a) is about 130 °C to about 175 °C.

In some embodiments, step (a) is maintained at the elevated temperature for about 5 minutes to about 240 minutes.

In some embodiments, the volatile alcohol in step (a) pretreatment is ethanol.

In some embodiments, the pretreatment liquor comprises about 1 weight % to about 50 weight % sulfur dioxide, about 10 weight % to about 80 weight % volatile alcohol, and less than 89 weight % water (including water from the steamed lignocellulosic biomass).

In some embodiments, the pretreatment liquor comprises about 5 weight % to about 20 weight % sulfur dioxide, about 20 weight % to about 50 weight % volatile alcohol, and about 30 weight % to about 75 weight % water.

In some embodiments, the ratio of pretreatment liquor to lignocellulosic biomass, based on dry weight of the lignocellulosic biomass, is about 1 kg/kg to 12 kg/kg (including water from the steamed lignocellulosic biomass in the mass of the pretreatment liquor).

In some embodiments, the step (a) pretreatment is performed in a continuous mode. In other embodiments, the step (a) pretreatment is performed in a batch mode.

In some embodiments, the step (b1) recovery of free sulfur dioxide and alcohol is performed using one or more of a blow tank, a distillation column, a fractionating column and a stripper. A sequence of steps can be used.

In some embodiments, the first mixture in step (c) before adjusting the pH is retained at an elevated temperature for about five minutes to about 15 hours. In various embodiments, the elevated temperature is about 80 °C to about 130 °C.

In some embodiments, the step (d) enzymatic hydrolysis is performed in a continuous mode. In other embodiments, step (d) enzymatic hydrolysis is performed in a batch mode. In some embodiments, the step (d) enzymatic hydrolysis can be performed sequentially in two or more steps, for example, such steps can be liquefaction and saccharification. Some or all of steps can be batch, fed batch, or continuous.

In some embodiments, the step (d) enzymatic hydrolysis can be performed at high total solids of >9%, for example, 10%, 11%, 12%, 13%, 14%, 15%, 16%, 17%, 18%, 19%, 20%, 21%, 22%, 23%, 24%, 25%, 26%, 27%, 28%, 29%, 30%, or higher.

In some embodiments, the step (d) enzymatic hydrolysis can be performed such that the whole amount of the pretreated material and the whole amount of enzyme are added at the beginning of enzyme hydrolysis.

In some embodiments, the step (d) enzymatic hydrolysis can be performed such that the pretreated material is added to enzyme hydrolysis in batches or stepwise, while the enzyme is added either in batches, stepwise, or the whole amount of the enzyme is added at once.

In some embodiments, the step (d) enzymatic hydrolysis can be performed such that the enzyme is added to enzyme hydrolysis in batches or stepwise, while the pretreated material is added in batches, stepwise or the whole amount of the pretreated material is added at once.

In all embodiments, there is no separation of the cellulose from the remainder of the first mixture between step (b1) and step (d). In other words, cellulose and precipitated lignin are both present in the mixture undergoing enzymatic hydrolysis in step (d). The lack of a cellulose separation step reduces the equipment and energy required to run the process.

In some embodiments, in step (d) enzymatic hydrolysis is performed with an enzyme charge of less than 5 FPU, less than 4 FPU, less than 3.5 FPU, less than 3 FPU, less than 2.5 FPU, less than 2 FPU or 0.2 to 5 FPU, 0.5 to 3.5 FPU, 0.5 to 3.3 FPU, 0.5 to 2.5 FPU, 0.5 to 2 FPU, or 0.8 to 1.6 FPU per o.d. g biomass.

In some embodiments, lignin, alkoxylated lignin and/or lignosulfonate is/are partly or wholly separated in step (d2), in step (e2) or in step (f2).

In some embodiments, the first mixture after step (b), the second mixture after step (d), or both, are subjected to pH adjustment to a pH of 7-11 and maintaining for 10-120 minutes at 20-100 °C, before enzyme hydrolysis step (d), before fermentation step (e), or before both steps (d) and (e), followed by pH adjustment to a pH of less than 7.

In some embodiments, residual sulfur dioxide is removed from the first mixture after step (b), from the second mixture after step (d), or from both, by adding one or more aqueous solutions comprising hydrogen peroxide and/or by adding air or steam.

In some embodiments, the lignocellulosic biomass is hardwood, softwood or herbaceous biomass. In some embodiments, the lignocellulosic biomass is softwood. In some embodiments, the lignocellulosic biomass is hardwood. In some embodiments, the lignocellulosic biomass is herbaceous biomass. In further embodiments, the process can be carried out with mixed lignocellulosic biomass, e.g., a combination of one or more of softwood, hardwood, and herbaceous biomass. For example, two or more hardwood and/or softwood species can be processed simultaneously using the same equipment and the same or similar process conditions.

Process 1 may be configured such that sugar and lignin dealkoxylation may not occur or may not be complete in step (b1), for example, due to low temperature and/or low retention time. The extent of the reactions is adjusted by changing conditions of step (b1). If desired, the dealkoxylation reactions may be performed during enzymatic hydrolysis step (d). In some embodiments, no or limited lignin dealkoxylation is performed, to produce alkoxylated lignin. Process 1 pretreatment conditions may be configured to minimize the production of alkoxylated sugars and/or alkoxylated lignin.

### Process 2.

Process 2 is similar to Process 1, but it has an additional step, step (b2), wherein dealkoxylation reactions are carried out by heat treatment. Process 2 is favored in some situations, for example, when complete or near complete dealkoxylation of lignin and/or sugars is desired. Lignin dealkoxylation may be preferred for certain downstream applications. Also, or alternatively, if maximizing sugar fermentation yields is important, then the sugar dealkoxylation of Process 2 may be preferred over the streamlined methods of Process 1. The lignin and sugar dealkoxylation of Process 2 also provides higher pretreatment alcohol recovery rates.

In a second embodiment, referred to herein as Process 2, this disclosure provides a process for the production of one or more fermentation products and lignin from lignocellulosic biomass comprising:
(a) contacting lignocellulosic biomass or steamed lignocellulosic biomass with a pretreatment liquor at an elevated temperature;
   wherein the pretreatment liquor comprises sulfur dioxide, volatile alcohol, and water;
   thereby releasing and/or dissolving lignin and hemicelluloses from the lignocellulosic biomass into the pretreatment liquor, resulting in a mixture comprising lignin and hemicelluloses dissolved in the pretreatment liquor and a solid fraction comprising mainly cellulose from the lignocellulosic biomass, which mixture is referred to as the pretreated material;
(b1) recovering sulfur dioxide and alcohol, thereby precipitating lignin, producing a first mixture comprising cellulose, lignin, and monosaccharides;
(b2) retaining the first mixture at an elevated temperature and at a desired pressure and mixing rate or recirculating rate, whereby volatile alcohol and optionally sulfur dioxide chemically or loosely bound to one or more of sugars (for example, in alkyl glycosides and α-hydroxysulfonic acids), lignin (for example, in alkoxylated lignin) and other compounds (for example, in esters and acetals), is/are released/split off and optionally recovered, and oligosaccharides and polysaccharides are optionally hydrolyzed, thereby liberating monosaccharides and optionally dealkoxylating lignin, providing a second mixture comprising cellulose, lignin, and monosaccharides;
(c) adjusting the pH of the second mixture to an appropriate level for enzymatic hydrolysis, for example, 4 to 7, 4.5 to 6.5, or 5 to 6;
(d) contacting the pH-adjusted second mixture with a cellulase, a glucosidase, a hemicellulase, or a combination thereof, to provide a third mixture comprising glucose derived from cellulose and monosaccharides derived from hemicellulose as well as lignin;
(d2) optionally removing lignin, alkoxylated lignin and/or lignosulfonate from the third mixture providing fourth mixture;
(e) subjecting the third or fourth mixture to fermentation, optionally in combination with adjusting the pH, for example, to 3.5 to 7, to 4.5 to 6.5, or to about 5 to about 6, to produce fermented broth containing one or more fermentation products;
(e2) optionally removing lignin, alkoxylated lignin and/or lignosulfonate from the fermented broth;
(f) separating the one or more fermentation products, optionally using a beer column, to provide a fermentation product-lean mixture (for example, stillage); and
(f2) optionally removing lignin, alkoxylated lignin and/or lignosulfonate from the fermentation product-lean mixture.

In some embodiments, the elevated temperature of step (a) is about 130 °C to about 175 °C.

In some embodiments, step (a) is maintained at the elevated temperature for about 5 minutes to about 240 minutes.

In some embodiments, the volatile alcohol in step (a) pretreatment is ethanol.

In some embodiments, the pretreatment liquor comprises about 1 weight % to about 50 weight % sulfur dioxide, about 10 weight % to about 80 weight % volatile alcohol, and less than 89 weight % water (including water from the steamed lignocellulosic biomass).

In some embodiments, the pretreatment liquor comprises about 5 weight % to about 20 weight % sulfur dioxide, about 20 weight % to about 50 weight % volatile alcohol, and about 30 weight % to about 75 weight % water.

In some embodiments, the ratio of pretreatment liquor to lignocellulosic biomass, based on dry weight of the lignocellulosic biomass, is about 1 kg/kg to about 12 kg/kg (including water from the steamed lignocellulosic biomass in the mass of the pretreatment liquor).

In some embodiments, the step (a) pretreatment is performed in a continuous mode. In other embodiments, the step (a) pretreatment is performed in a batch mode.

In some embodiments, the step (b1) recovery of free sulfur dioxide and alcohol is performed using one or more of a blow tank, a distillation column, a fractionating column and a stripper. A sequence of the steps can be used.

In some embodiments, the retention of pretreated material in step (b2) is accomplished in a tank or a series of tanks equipped with heated recirculation line(s) and optionally one or more of a distillation column, fractionating column, and stripper. The column may be constructed with trays, packing, or a combination of each, depending on the chemicals to be removed. Transferring pretreated material to said tank can be accomplished by pressure difference (blow), pumping and other means.

In some embodiments, step (b2) is carried out in a tank or a series of tanks, for a desired time, for example, 0.1, 0.5, 1, 1.5, 2, 2.5, 3, 3.5, 4, 5, 6, 7, 8, 9, 10, 11, 12, 13, 14 or 15 hours, at a desired temperature, for example, 50, 60, 70, 75, 80, 85, 90, 95, 97.5, 100, 102.5, 105, 107.5, 110, 112.5, 115, 117.5, 120, 122.5, or 125 °C, and at a resulting or desired pressure, whereby alcohol and optionally sulfur dioxide chemically bound or loosely bound to sugars, lignin and other compounds, is/are released and optionally recovered, while one or more of alkoxylated lignin and alkyl glycosides (alkoxylated sugars) are optionally dealkoxylated, and oligosaccharides are optionally hydrolyzed to monosaccharides. The amount of alcohol release can be controlled by the temperature and reaction time.

In some embodiments, in step (b2), free alcohol, optionally sulfur dioxide and optionally some of the pretreatment by-products including but not limited to acetic acid, formic acid, methanol, alkyl acetates, furfural, are partly or wholly removed from the pretreated material and concentrated in a desired way.

In some embodiments, the step (d) enzymatic hydrolysis is performed in a continuous mode. In other embodiments, the step (d) enzymatic hydrolysis is performed in a batch mode.

In some embodiments, the step (d) enzymatic hydrolysis can be performed sequentially in two or more steps, for example, such steps can be liquefaction and saccharification. Some or all these steps can be carried out in batch or continuous modes of operation.

In some embodiments, the step (d) enzymatic hydrolysis can be performed at high total solids of >9%, for example, 10%, 11%, 12%, 13%, 14%, 15%, 16%, 17%, 18%, 19%, 20%, 21%, 22%, 23%, 24%, 25%, 26%, 27%, 28%, 29%, 30% or higher.

In some embodiments, the step (d) enzymatic hydrolysis can be performed such that the whole amount of the pretreated material and the whole amount of enzyme are added at the beginning of enzyme hydrolysis.

In some embodiments, the step (d) enzymatic hydrolysis can be performed such that the pretreated material is added to enzyme hydrolysis in batches or stepwise, while the enzyme is added either in batches, stepwise, or the whole amount of the enzyme is added at once.

In some embodiments, the step (d) enzymatic hydrolysis can be performed such that the enzyme is added to enzyme hydrolysis in batches or stepwise, while the pretreated material is added in batches, stepwise or the whole amount of the pretreated material is added at once.

In all embodiments, there is no separation of the cellulose from the remainder of the first or second mixture between step (b1) and step (d), or between step (b2) and step (d). In other words, cellulose and precipitated lignin are both present in the mixture undergoing enzymatic hydrolysis in step (d). The lack of a cellulose separation step reduces the equipment and energy required to run the process.

In some embodiments, in step (d) enzymatic hydrolysis is performed with an enzyme charge of less than 5 FPU, less than 4 FPU, less than 3.5 FPU, less than 3 FPU, less than 2.5 FPU, less than 2 FPU or 0.2 to 5 FPU, 0.5 to 3.5 FPU, 0.5 to 3.3 FPU, 0.5 to 2.5 FPU, 0.5 to 2 FPU, or 0.8 to 1.6 FPU per o.d. g biomass.

In some embodiments, lignin, alkoxylated lignin and/or lignosulfonate is/are is partly or wholly separated in step (d2), in step (e2), or in step (f2).

In some embodiments, alkoxylated lignin is partially or wholly dealkoxylated in step (b1) and/or step (b2), and the resulting lignin material is partly or wholly separated in step (d2), in step (e2), or in step (f2).

In some embodiments, the second mixture after step (b2), the third mixture after step (d), or both, are subjected to pH adjustment to a pH of 7-11 and maintaining for 10-120 minutes at 20-100 °C, before enzyme hydrolysis step (d), before fermentation step (e), or before both steps (d) and (e), followed by pH adjustment to a pH of less than 7.

In some embodiments, residual sulfur dioxide is removed from the second mixture after step (b2), from the third mixture after step (d), or from both, by adding one or more aqueous solutions comprising hydrogen peroxide and/or by adding air or steam.

In some embodiments, the lignocellulosic biomass is hardwood, softwood or herbaceous biomass. In some embodiments, the lignocellulosic biomass is softwood. In some embodiments, the lignocellulosic biomass is hardwood. In some embodiments, the lignocellulosic biomass is herbaceous biomass. In further embodiments, the process can be carried out with mixed lignocellulosic biomass, e.g., a combination of one or more of softwood, hardwood, and herbaceous biomass. For example, two or more hardwood and/or softwood species can be processed simultaneously using the same equipment and the same or similar process conditions.

### Process 1 and Process 2.

In some embodiments, the volatile alcohol is methanol, ethanol, 1-propanol, 2-propanol, 2-methyl-2-propanol, or a mixture thereof. In one preferred embodiment, the volatile alcohol is ethanol.

In some embodiments, instead of or in addition to volatile alcohol or alcohols, one or more volatile hydrocarbons (such as one or more of pentane, hexane, cyclopentane, and cyclohexane); ethers (such as one or more of diethyl ether and methyl tert-butyl ether (MTBE)), or mixtures thereof, is or are utilized.

In some embodiments, the recovering of sulfur dioxide in step (b1) (Process 1), or in step (b1) and optionally step (b2) (Process 2) comprises:
(i) releasing gaseous vapors from the pretreated material at elevated temperature and/or at a lower pressure, wherein the gaseous vapors released from the pretreated material comprise sulfur dioxide and one or more of water vapor and alcohol vapor;
(ii) condensing at least a portion of the gaseous vapors to provide sulfur dioxide gas and a liquid containing at least one of water and alcohol;
(iii) optionally purifying the sulfur dioxide gas by removing traces of water vapor and organic volatiles;
(iv) liquefying sulfur dioxide gas by pressure change and/or temperature reduction of the sulfur dioxide gas, thereby providing liquid sulfur dioxide; and optionally storing the liquid sulfur dioxide; and
(v) introducing the liquid sulfur dioxide to the container of step (a) or another pretreatment vessel, optionally by pumping.

In some embodiments, ethanol is used as the volatile alcohol and it is obtained in step (ii), condensed and used in a desired way or ways which are listed as optional: 1) recycled back to pretreatment; and/or 2) combined with fermentation broth and recovered together with the ethanol produced in fermentation.

In some embodiments, one or more of water, phosphoric acid, hydrochloric acid, sulfuric acid, nitric acid, acetic acid, formic acid, oxalic acid, and process condensates are added to step (b1) (Process 1), or step (b1) and/or step (b2) (Process 2).

In some embodiments, one or both of step (b1) of Process 1 or 2, and step (b2) of Process 2, are accomplished by retaining and recirculating the pretreated material in a tank equipped with heated recirculation line and a distillation column, a fractionating column, or a stripper, for a period of time, thereby removing volatile chemical compounds (for example, volatile alcohol, sulfur dioxide and pretreatment by-products) from the pretreated material by flashing, and the volatile chemical compounds are obtained by passing the vapor through the distillation column, fractionating column, or stripper; thereby removing the volatile chemical compounds from the pretreated material, and concentrating and recovering the volatile chemical compounds. The volatile pretreatment by-products may include, but are not limited to, acetic acid, formic acid, methanol, ethyl acetate, and furfural.

In some embodiments, ethanol produced in fermentation is separated by distillation.

In some embodiments, alkoxylated and/or non-alkoxylated lignin fractions are separated after removal of volatile alcohol (typically ethanol), for example, by centrifugation or using a filter press, or both.

In some embodiments, lignosulfonate is produced and can be separated if desired at various points of the process, for instance, after enzymatic hydrolysis, after fermentation or after removal of volatile alcohol (typically ethanol). Lignosulfonate can be separated, for example, by membrane filtration.

In some embodiments, prior to step (a), the lignocellulosic biomass is contacted with steam, wherein the lignocellulosic biomass absorbs water from the steam, removing air and optionally turpentine from the lignocellulosic biomass. Contacting the biomass with steam can be carried out in a first container, such as a steaming bin. The biomass can then be transferred to a separate container for carrying out pretreatment step (a).

In various embodiments, a distillation column, fractionating column or a stripper and a condenser are used to facilitate the separation of sulfur dioxide and volatile alcohol components.

In one specific embodiment of Process 1, the invention provides a process for the production of one or more fermentation products and lignin comprising:
(a) contacting the steamed lignocellulosic biomass with a pretreatment liquor at a temperature between about 145 °C and about 175 °C under pressure for 5 to 60 minutes;
   wherein the pretreatment liquor comprises 3 to 30 weight % sulfur dioxide, 10-50 weight % ethanol, and 20 to 87 weight % water (including water from the steamed lignocellulosic biomass);
   thereby releasing and/or dissolving lignin and hemicelluloses from the lignocellulosic biomass into the pretreatment liquor, resulting in a mixture comprising lignin, lignosulfonic acid (LS) and hemicelluloses dissolved in the pretreatment liquor and a solid fraction comprising mainly cellulose from the lignocellulosic biomass, which mixture is referred to as the pretreated material;
(b1) recovering sulfur dioxide and ethanol, thereby precipitating lignin, producing a first mixture comprising cellulose, lignin, lignosulfonic acid (LS), and monosaccharides;
(c) adjusting the pH of the second mixture to between about 4.8 and about 5.8 for enzymatic hydrolysis;
(d) contacting the pH-adjusted second mixture with a combination of cellulases, glucosidases and hemicellulases at a temperature of about 50 °C to about 60 °C, to provide a third mixture comprising glucose derived from cellulose and monosaccharides derived from hemicelluloses as well as lignin and lignosulfonate (salt of lignosulfonic acid); and
(e) subjecting the third mixture to fermentation to produce one or more fermentation products, optionally in combination with adjusting pH of the third mixture. A preferred fermentation product is ethanol. However, the process can provide other fermentation products, including a set of fermentation products that are primarily or exclusively products other than ethanol.

In one specific embodiment of Process 2, the invention provides a process for the production of one or more fermentation products and lignin comprising:
(a) contacting the steamed lignocellulosic biomass with a pretreatment liquor at a temperature between about 145 °C and about 175 °C under pressure for 5 to 60 minutes;
   wherein the pretreatment liquor comprises 3 to 30 weight % sulfur dioxide, 30-60 weight % ethanol, and 10 to 67 weight % water (including water from the steamed lignocellulosic biomass);
   thereby releasing and/or dissolving lignin and hemicelluloses from the lignocellulosic biomass into the pretreatment liquor, resulting in a mixture comprising lignin, lignosulfonic acid (LS) and hemicelluloses dissolved in the pretreatment liquor and a solid fraction comprising mainly cellulose from the lignocellulosic biomass, which mixture is referred to as the pretreated material;
(b1) recovering sulfur dioxide and ethanol, thereby precipitating lignin, producing a first mixture comprising cellulose, lignin, lignosulfonic acid (LS), and monosaccharides;
(b2) retaining the first mixture for 0.1 to 8 hours at a temperature of about 90-130 °C, at desired pressure and mixing rate or recirculating rate, whereby ethanol and optionally sulfur dioxide chemically or loosely bound to sugars, lignin and other compounds, is/are released/split off and optionally recovered, and oligosaccharides and polysaccharides are optionally hydrolyzed, thereby liberating monosaccharides, providing a second mixture, wherein the second mixture comprises less than 10 kg per BD tonne wood of furfural, hydroxymethyl furfural, and levulinic acid combined;
(c) adjusting the pH of the second mixture to between about 5 and about 6 for enzymatic hydrolysis;
(d) contacting the pH-adjusted second mixture with a combination of cellulases, glucosidases and hemicellulases at a temperature of about 50 °C to about 60 °C, to provide a third mixture comprising glucose derived from cellulose and monosaccharides derived from hemicelluloses as well as lignin and lignosulfonate (salt of lignosulfonic acid); and
(e) subjecting the third mixture to fermentation, optionally in combination with adjusting pH of the third mixture, to produce one or more fermentation products. A preferred fermentation product is ethanol. However, the process can provide other fermentation products, including a set of fermentation products that are primarily or exclusively products other than ethanol.

In some embodiments of the process, ethanol obtained in step (b1) and optionally step (b2) is condensed and recycled back to pretreatment; and/or at least partly combined with fermentation broth and recovered together with the ethanol produced in fermentation. In various embodiments, in steps (b1) and/or (b2) one or more of the pretreatment by-products including but not limited to acetic acid, formic acid, methanol, ethyl acetate, furfural are partly or wholly removed from the pretreated material and concentrated in the desired way.

As will be readily understood by one of skill in the art, in both Process 1 and Process 2, the pH of compositions to undergo enzymatic hydrolysis or fermentation is typically adjusted to appropriate hydrolysis or fermentation pH levels, for example, to a pH as recited herein for hydrolysis or fermentation steps, to provide suitable conditions for the hydrolysis or fermentation.

In some embodiments, the lignocellulosic biomass is derived from softwoods. Achieving a high yield of saccharification products is more difficult from softwood lignocellulosic biomass (for example, pine, spruce, Douglas fir, larch, cedar, etc.) than from hardwood and herbaceous lignocellulosic biomass. The processes described herein can provide high-yields of 1) cellulose-derived fermentable glucose and 2) hemicellulose-derived fermentable monomeric sugars from all types of biomass including softwood-derived lignocellulosic biomass simultaneously in an economical manner without producing sticky lignin precipitates, wherein the combined yields for softwoods are about 76-83% or higher based on available polysaccharides in the biomass, at enzyme charge of 3.3 FPU/o.d. g biomass or lower. This yield is equivalent to or greater than 540-600 kg monosugars per BD metric tonne of softwood biomass containing 650 kg polysaccharides (anhydro) per BD tonne. The combined yields for hardwoods (for example, poplar, birch, beech, eucalyptus, aspen, maple, oak), agricultural wastes (for example, corn stover, wheat straw, sugarcane straw, empty fruit bunches, etc.) and energy crops (for example, energy cane) are about 81-85% or higher based on available polysaccharides in the biomass, at enzyme charge of 0.8 FPU/o.d. g biomass or lower, and about 83-93% or higher based on available polysaccharides in the biomass, at enzyme charge of 1.6 FPU/o.d. g biomass or lower. This yield is equivalent to or greater than 505-580 BD kg monosugars per BD metric tonne of corn stover biomass containing 555 kg polysaccharides (anhydro) per BD tonne.

The enzymatic digestibility of cellulose produced in this process is high, i.e., glucan-to-glucose conversion is over 80%, over 85%, over 90%, or over 95% at low enzyme dosage, such as, for softwoods, of about 3.3 FPU per o.d. g biomass or less, or, for hardwoods and herbaceous biomass, of about 1.6 FPU per o.d. g biomass, or about 0.8 FPU per o.d. g biomass, or less. The corresponding values for hemicelluloses conversion to monomeric sugars are over 65%, over 70%, over 75%, over 80%, over 85%, over 90% or over 95%. If present, alkyl glycosides are optionally converted to monosaccharides by enzymes (step (d) in Process 1) or optionally by retention of the slurry (the first mixture) at elevated temperature (step (b2) in Process 2). The sugars are readily fermentable without detoxification, which may be due to the low sugar and lignin degradation in the process.

Surprising is the fact that, despite the presence of relatively high amounts of water-insoluble lignin (in alkoxylated and/or non-alkoxylated forms), the enzymatic hydrolysis proceeds well with high glucose yields at low enzyme charges (such as 0.8 FPU per o.d. g biomass or less, or 1.6 FPU per o.d. g biomass or less). Surprising also is that after pretreatment, the whole slurry without detoxification can be enzymatically hydrolyzed at low enzyme charges with high yields of monomeric sugars (for example, 83% or higher for softwoods, and 93% or higher for herbaceous biomass, based on available in biomass). This innovation therefore eliminates the requirement of pulp washing.

In one preferred embodiment, this disclosure provides a process for the production of ethanol and lignin from softwood lignocellulosic biomass. The softwood lignocellulosic biomass can be contacted with a pretreatment liquor at about 150 °C (± 5 °C) under pressure to provide a mixture comprising lignin and hemicelluloses dissolved in the pretreatment liquor and a solid fraction comprising mainly cellulose. The pretreatment liquor can comprise about 7-15% sulfur dioxide, about 42-46.5% ethanol, and about 43-46.5% water. In one preferred embodiment, the pretreatment liquor comprises 5-9% sulfur dioxide, preferably 6-8% sulfur dioxide. In another preferred embodiment, the pretreatment liquor comprises 13-17% sulfur dioxide, preferably 14-16% sulfur dioxide. The pH of the first mixture comprising cellulose, lignin, and monosaccharides can be adjusted to an appropriate level for enzymatic hydrolysis, for example, a pH of about 5 to about 6, preferably about 5.5. Contacting the first mixture with one or more enzymes provides a second mixture comprising glucose, other monosaccharides, and lignin, wherein the total monomeric sugar content in the second mixture is 79% or higher, based on the available polysaccharides in softwood lignocellulosic biomass. The process can then be completed as described above for Process 1.

In one preferred embodiment, this disclosure provides a process for the production of ethanol and lignin from softwood lignocellulosic biomass comprising (a) contacting softwood lignocellulosic biomass with a pretreatment liquor at about 150 °C (± 5 °C) under pressure; wherein the pretreatment liquor comprises about 7-15% sulfur dioxide, about 42-46.5% ethanol, and about 43-46.5% water. In one preferred embodiment, the pretreatment liquor comprises 5-9% sulfur dioxide, preferably 6-8% sulfur dioxide. In another preferred embodiment, the pretreatment liquor comprises 13-17% sulfur dioxide, preferably 14-16% sulfur dioxide. In step (b1), sulfur dioxide and ethanol is preferably recovered, which results in the precipitation of lignin, producing a first mixture comprising cellulose, lignin, and monosaccharides. In step (b2), the first mixture is preferably retained at about 99 °C to about 120 °C at a suitable pressure and mixing rate or recirculating rate, whereby ethanol and optionally sulfur dioxide chemically or loosely bound to one or more of sugars (for example, in ethyl glycosides and α-hydroxysulfonic acids), lignin (for example, in ethoxylated lignin) and other compounds (for example, in esters and acetals), is/are released/split off and optionally recovered, and oligosaccharides and polysaccharides are optionally hydrolyzed, thereby liberating monosaccharides and optionally de-ethoxylating lignin, providing a second mixture. In step (c) the pH of the second mixture is adjusted to an appropriate level for enzymatic hydrolysis, preferably a pH of about 5 to about 6. In step (d), the pH-adjusted second mixture is contacted with a cellulase, a glucosidase, a hemicellulase, or a combination thereof, to provide a third mixture comprising glucose derived from cellulose and monosaccharides derived from hemicellulose as well as lignin; wherein total monomeric sugar content in the second mixture is 80% or higher, based on the available polysaccharides in softwood lignocellulosic biomass. The process can then be completed as described above for Process 2.

Surprising also is the fact that some biomass species, including pine wood, which is not suitable for acid sulfite pulping, can be utilized in the presently disclosed process.

Furthermore, the pretreatment surprisingly does not require explosion of the fiber structure to achieve high monosaccharide yields, contrary to the present state of the art. Accordingly, in some embodiments, the processes described herein exclude explosion of the fiber structure after pretreatment.

The invention provides, in one preferred embodiment, a process for the production of one or more fermentation products and lignin from herbaceous biomass/agricultural residues (e.g., corn stover) comprising:
(a) contacting herbaceous lignocellulosic biomass or steamed herbaceous lignocellulosic biomass with a pretreatment liquor at about 140-150 °C under pressure;
   wherein the pretreatment liquor comprises about 3-7% sulfur dioxide, about 23-24% ethanol, and about 69-74% water;
   thereby releasing and/or dissolving lignin and hemicelluloses from the lignocellulosic biomass into the pretreatment liquor, resulting in a mixture comprising lignin and hemicelluloses dissolved in the pretreatment liquor and a solid fraction comprising mainly cellulose from the lignocellulosic biomass, which mixture is referred to as the pretreated material;
(b1) recovering sulfur dioxide and alcohol, thereby precipitating lignin, producing a first mixture comprising cellulose, lignin, and monosaccharides;
(c) adjusting the pH of the first mixture to an appropriate level for enzymatic hydrolysis, for example, to about pH 5 to about pH 6;
(d) contacting the pH-adjusted first mixture with a cellulase, a glucosidase, a hemicellulase, a glycosidase or a combination thereof, to provide a second mixture comprising glucose derived from cellulose and monosaccharides derived from hemicelluloses as well as lignin;
wherein monomeric sugar content in the second mixture is 86% or higher, based on the available polysaccharides in herbaceous lignocellulosic biomass. The process can then be completed as described above for Process 1.

In another preferred embodiment, this disclosure provides a process for the production of one or more fermentation products and lignin from herbaceous lignocellulosic biomass/agricultural residues (e.g., corn stover) comprising:
(a) contacting herbaceous lignocellulosic biomass or steamed herbaceous lignocellulosic biomass with a pretreatment liquor at about 150 °C under pressure;
   wherein the pretreatment liquor comprises about 5% sulfur dioxide, about 47.5% ethanol, and about 47% water;
   thereby releasing and/or dissolving lignin and hemicelluloses from the lignocellulosic biomass into the pretreatment liquor, resulting in a mixture comprising lignin and hemicelluloses dissolved in the pretreatment liquor and a solid fraction comprising mainly cellulose from the lignocellulosic biomass, which mixture is referred to as the pretreated material;
(b1) recovering sulfur dioxide and ethanol, thereby precipitating lignin, producing a first mixture comprising cellulose, lignin, and monosaccharides;
(b2) retaining the first mixture at about 99 °C to about 120 °C and at a desired pressure and mixing rate or recirculating rate, whereby ethanol and optionally sulfur dioxide chemically or loosely bound to one or more of sugars (for example, in ethyl glycosides and α-hydroxysulfonic acids), lignin (for example, in ethoxylated lignin) and other compounds (for example, in esters and acetals), is/are released/split off and optionally recovered, and oligosaccharides and polysaccharides are optionally hydrolyzed, thereby liberating monosaccharides and optionally de-ethoxylating lignin, providing a second mixture;
(c) adjusting the pH of the second mixture to an appropriate level for enzymatic hydrolysis, for example, to about pH 5 to about pH 6;
(d) contacting the pH-adjusted second mixture with a cellulase, a glucosidase, a hemicellulase, or a combination thereof, to provide a third mixture comprising glucose derived from cellulose and monosaccharides derived from hemicellulose as well as lignin;
wherein monomeric sugar content in the second mixture is 85% or higher, based on the available polysaccharides in herbaceous lignocellulosic biomass. The process can then be completed as described above for Process 2.

Furthermore, hardwood biomass (angiosperm) is considerably easier to pretreat than softwood biomass (gymnosperm), such that the efficiency of its pretreatment efficiency lies generally between that of softwood and herbaceous (angiosperm) biomass. Accordingly, the processes described herein can be carried out on hardwood biomass to provide monosugar yields equal to or greater than the softwood (gymnosperm) yields described herein, at similar enzyme charges, and wherein the monosugar yields can approach the yields described herein for herbaceous (non-woody) biomass (angiosperms) such as corn stover, at similar enzyme charges.

Figures 1-5 illustrate flow diagrams of the processes described herein.

Figure 1 illustrates Processes 1 and 2. The step (a) Pretreatment, steps (b1) and (b2) Chemicals recovery, step (c) pH adjustment prior to Enzymatic hydrolysis, step (d) Enzymatic hydrolysis, optional step (d2) Separation of lignin, alkoxylated lignin and/or lignosulfonate prior to fermentation, step (e) Fermentation, optional step (e2) Separation of lignin, alkoxylated lignin and/or lignosulfonate after fermentation, step (f) Fermentation product(s) separation and optional step (f2) Separation of lignin, alkoxylated lignin and/or lignosulfonate after fermentation product(s) separation, are shown.

Lignin, alkoxylated lignin, or mixtures of both, can be obtained by both Processes 1 and 2 by adjusting pretreatment conditions as well as by adjusting conditions of the step (b2) in Process 2. One or more of lignin, alkoxylated lignin, mixtures of both, and lignosulfonate, can be separated in a desired point of the process, for example, before fermentation (optional step (d2)), after fermentation (optional step (e2)), or after fermentation product(s) separation (optional step (f2)).

Figure 2 provides an example of flow diagram when **softwood** biomass is used as feedstock and Process 1 is used.

Figure 3 provides an example of flow diagram when **softwood** biomass is used as feedstock and Process 2 is used.

Figure 4 provides an example of flow diagram when **corn stover** biomass is used as feedstock and Process 1 is used.

Figure 5 provides an example of flow diagram when **corn stover** biomass is used as feedstock and Process 2 is used.

Figure 6 provides a comparison of the monosugar yields obtained by the methods described by Example 2 to those obtained in the conventional hot water (hydrothermal) pretreatment of corn stover (Example 3). It is noteworthy that the hot water process provides lower sugar yield compared to the processes described herein, at all parameters tested. The hot water process does not reach the yields of the processes described herein even at much higher enzyme charges. A comparison of the enzyme hydrolysis monosaccharide yields for the process described herein and conventional hot water pretreatment at an enzyme charge of 1.6 FPU/o.d. g biomass shows the improvement of enzyme hydrolysis monosaccharide yield from an average of 54% to an average of 90%, i.e., a significant improvement of approximately 67%. Furthermore, at an enzyme charge of 6.6 FPU/o.d. g biomass, conventional hot water process results in an average monosaccharide yield of 71%, while the process described herein results in an average monosaccharide yield of 83% at an enzyme charge of only 0.8 FPU/o.d. g biomass, i.e., showing 17% higher enzyme hydrolysis monosaccharide yield at 8-fold lower enzyme dosage.

As described herein, one or more of lignin, alkoxylated lignin, and lignosulfonate, can be removed midstream from Process 1 or Process 2 at different points of the process. See Figures 1-5. However, the removal of lignin, alkoxylated lignin, and lignosulfonate is optional, and is not required for high monosaccharide yields in enzyme hydrolysis and is not required for high fermentation product yields.

### Definitions.

The following definitions are included to provide a clear and consistent understanding of the specification and claims. As used herein, the recited terms have the following meanings. All other terms and phrases used in this specification have their ordinary meanings as one of skill in the art would understand. Such ordinary meanings may be obtained by reference to technical dictionaries, such as *Hawley's Condensed Chemical Dictionary* 14^{th} Edition, by R.J. Lewis, John Wiley & Sons, New York, N.Y., 2001.

References in the specification to "one embodiment", "an embodiment", etc., indicate that the embodiment described may include a particular aspect, feature, structure, moiety, or characteristic, but not every embodiment necessarily includes that aspect, feature, structure, moiety, or characteristic. Moreover, such phrases may, but do not necessarily, refer to the same embodiment referred to in other portions of the specification. Further, when a particular aspect, feature, structure, moiety, or characteristic is described in connection with an embodiment, it is within the knowledge of one skilled in the art to affect or connect such aspect, feature, structure, moiety, or characteristic with other embodiments, whether or not explicitly described.

The singular forms "a," "an," and "the" include plural reference unless the context clearly dictates otherwise. Thus, for example, a reference to "a compound" includes a plurality of such compounds, so that a compound X includes a plurality of compounds X. It is further noted that the claims may be drafted to exclude any optional element. As such, this statement is intended to serve as antecedent basis for the use of exclusive terminology, such as "solely," "only," and the like, in connection with any element described herein, and/or the recitation of claim elements or use of "negative" limitations.

The term "and/or" means any one of the items, any combination of the items, or all of the items with which this term is associated. The phrases "one or more" and "at least one" are readily understood by one of skill in the art, particularly when read in context of its usage. For example, the phrase can mean one, two, three, four, five, six, ten, 100, or any upper limit approximately 10, 100, or 1000 times higher than a recited lower limit.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements. When values are expressed as approximations, by use of the antecedent "about," it will be understood that the particular value without the modifier "about" also forms a further aspect.

The term "about" can refer to a variation of ± 5%, ± 10%, ± 20%, or ± 25% of the value specified. For example, "about 50" percent can in some embodiments carry a variation from 45 to 55 percent, or as otherwise defined by a particular claim. For integer ranges, the term "about" can include one or two integers greater than and/or less than a recited integer at each end of the range. Unless indicated otherwise herein, the term "about" is intended to include values, e.g., weight percentages, proximate to the recited range that are equivalent in terms of the functionality of the individual ingredient, composition, or embodiment. The term about can also modify the endpoints of a recited range as discussed above in this paragraph.

The term "sticky lignin" refers to condensed lignin that precipitates from aqueous solutions and binds to surfaces of processing equipment, which leads to decreased performance of the equipment by plugging and/or clogging equipment valves and pipes. Precipitated sticky lignin hardens over time, eventually requiring cessation of operations to remove the precipitated sticky lignin from the equipment.

As will be understood by one skilled in the art, for any and all purposes, particularly in terms of providing a written description, all ranges recited herein also encompass any and all possible sub-ranges and combinations of sub-ranges thereof, as well as the individual values making up the range, particularly integer values. It is therefore understood that each unit between two particular units are also disclosed. For example, if 10 to 15 is disclosed, then 11, 12, 13, and 14 are also disclosed, individually, and as part of a range. A recited range (e.g., weight percentages or carbon groups) includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc. As will also be understood by one skilled in the art, all language such as "at least", "greater than", "less than", "more than", "or more", and the like, include the number recited and such terms refer to ranges that can be subsequently broken down into sub-ranges as discussed above. In the same manner, all ratios recited herein also include all sub-ratios falling within the broader ratio. Accordingly, specific values recited for radicals, substituents, and ranges, are for illustration only; they do not exclude other defined values or other values within defined ranges for radicals and substituents. It will be further understood that the endpoints of each of the ranges are significant both in relation to the other endpoint, and independently of the other endpoint.

One skilled in the art will also readily recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of any one or more of members of a recited group. Accordingly, provisos may apply to any of the disclosed categories or embodiments whereby any one or more of the recited elements, species, or embodiments, may be excluded from such categories or embodiments, for example, for use in an explicit negative limitation.

The following Examples are intended to further illustrate the above invention and should not be construed as to narrow its scope. One skilled in the art will readily recognize that the Examples suggest many other ways in which the invention could be practiced. It should be understood that numerous variations and modifications may be made while remaining within the scope of the invention.

### EXAMPLES

### Example 1. Pretreatment and enzyme hydrolysis of Scots pine

Scots pine sawdust (biomass) containing 7% moisture (as-received basis), 37.8% glucan (oven dry basis), 5.4% xylan, 2.6% galactan, 1.7% arabinan, 10.8% mannan, 1.2% acetyl groups, 23.4% lignin, 6.1% acetone extractives, and 0.6% ash was pretreated in rotating minireactors submerged in heated oil. A liquor for pretreatment was prepared resulting in a moisture-adjusted composition of 7-15% sulfur dioxide, 42-46.5% ethanol and 43-46.5% water, by weight, and the liquor was charged at about 4 parts liquor per 1 part of dry biomass (i.e., at liquor-to-solid ratio of 4 kg/kg). Deionized water was used. The biomass and liquor were combined, and the pretreatment was carried out under pressure at a final temperature of 150 °C, which final temperature was achieved about 10 minutes after initiation of the pretreatment. The heat applied to the pretreatment liquor and biomass results in a pressure increase within the closed system of the minireactor. The amount of time at the final temperature was 60 minutes; cooling down time was about 2 minutes (cooling by submerging the reactors in ice water).

Unreacted sulfur dioxide was removed slowly from the system by venting, and no fiber explosion was used. No separation of pulp and liquor took place. The material was transferred to a Rotary Evaporator flask and evaporated at about 75 °C for about 25 minutes with stepwise decrease in pressure from atmospheric to about 150 Torr, to provide a final total solids content of about 23-26%. To further remove ethanol, water was added and the evaporation cycle was repeated.

In the various experiments, the material was heat treated at various conditions (total solids ^{~}20-27%, a temperature 99 °C or 120 °C, for a duration of 1-8 hours), using either a Rotavap flask or minireactor submerged in oil bath.

In all the experiments, the material was transferred to a shake flask and diluted with deionized water. The pH of the mixture was adjusted by adding a 30% calcium hydroxide slurry to achieve a pH of 4, followed by the pH adjustment with a 15% ammonium hydroxide solution to achieve a pH of 5.3-5.5. The material was then subjected to enzymatic hydrolysis for 96 hours. A commercially available enzyme cocktail containing cellulases, hemicellulases and β-glucosidases was used. Total solids content during enzymatic hydrolysis in all experiments was about 12-13%, the pH was maintained at 4.8-5.5 using 15% ammonium hydroxide solution, and the temperature was maintained at about 53 °C. The shaking rate was 200 RPM.

**Tables 1 and 2** display the surprisingly high sugar yields at low enzyme charge of 3.3 FPU/o.d. g biomass. Overall glucose yields of 81-91% (based on the available glucan in the biomass) were obtained despite the presence of high amounts of water-insoluble lignin fractions that are generally inhibitory to enzyme hydrolysis. The overall hemicellulose sugar yield reached 68-74% (based on the available hemicelluloses in the biomass) which may be explained by the fact that the used enzyme cocktail was not optimized for softwood hemicelluloses (i.e., glucomannans). The overall monosugar yield achieved for pine was 76-83% (based on the available polysaccharides in the biomass) at 3.3 FPU/o.d. g biomass enzyme dosage. Further improvement of yield by optimizing enzyme cocktail or other means is expected.

The compositions were analyzed to measure the content of sugar degradation products - furfural, hydroxymethyl furfural and levulinic acid, the sum of which was found to account for less than 5 kg per BD tonne biomass (< 0.5%), which is unexpectedly low based on the relatively high pretreatment temperature (150 °C).

**Table 1. Sugar yields of Example 1 (7% sulfur dioxide).**

| Experiment | No. 1.1 | No. 1.2 | No. 1.3 | No. 1.4 | No. 1.5 | No. 1.6 | No. 1.7 |
|---|---|---|---|---|---|---|---|
| Moisture-adjusted pretreatment liquor composition, by weight | 7% sulfur dioxide, 46.5% ethanol, and 46.5% water | | | | | | |
| Heat Treatment | None | 4 hr @ 99°C and 20.3% total solids | 4 hr @ 99°C and 24.7% total solids | 8 hr @ 99°C and 23.7% total solids | 8 hr @ 99°C and 26.5% total solids | 1 h @ 120°C and 20.7% total solids | 1 h @ 120°C and 23.5% total solids |
| Enzyme charge, FPU per o.d. g biomass | 3.3 | 3.3 | 3.3 | 3.3 | 3.3 | 3.3 | 3.3 |
| Monosaccharide yield, % on available in biomass | | | | | | | |
| Glucose | 91 | 81 | 81 | 85 | 81 | 80 | 88 |
| Hemicellulose Sugars | 58 | 68 | 69 | 68 | 70 | 70 | 74 |
| Total | 79 | 78 | 76 | 80 | 76 | 76 | 83 |
| Monosaccharide yield, kg per BD tonne biomass | | | | | | | |
| Total | 513 | 506 | 493 | 519 | 493 | 493 | 539 |

**Table 2. Sugar yields of Example 1 (15% sulfur dioxide).**

| Experiment | 1.8 | 1.9 | 1.10 |
|---|---|---|---|
| Moisture-adjusted pretreatment liquor composition, by weight | 15% sulfur dioxide, 43% ethanol, and 42% water | | |
| Heat Treatment | 4 hr @ 99°C and 23.6% total solids | 8 hr @ 99°C and 22.1% total solids | 1 h @ 120°Cand 20.1% total solids |
| Enzyme charge, FPU per o.d. g biomass | 3.3 | 3.3 | 3.3 |
| Monosaccharide yield, % on available in biomass | | | |
| Glucose | 86 | 88 | 85 |
| Hemicellulose Sugars | 69 | 72 | 73 |
| Total | 80 | 82 | 81 |
| Monosaccharide yield, kg per BD tonne biomass | | | |
| Total | 519 | 532 | 526 |

### Example 2. Pretreatment and enzyme hydrolysis of corn stover

Corn Stover (biomass) containing 8% moisture (as-received basis), 33.7% glucan (oven dry basis), 18.2% xylan, 0.0% galactan, 3.2% arabinan, 0.4% mannan, 2.5% acetyl groups, 17.4% lignin, 1.8% acetone extractives, and 5.0% ash was pretreated in rotating minireactors submerged in heated oil. A liquor for pretreatment was prepared resulting in a moisture-adjusted composition that is provided in Tables 3 and 4. The liquor was charged at about 5.5 parts liquor per 1 part of dry biomass (i.e., at liquor-to-solid ratio of 5.5 kg/kg). Laboratory Type II water was used. The biomass and liquor were combined, and the pretreatment was carried out at a final temperature of 140-155 °C, which final temperature was achieved about 10 minutes after initiation of the pretreatment. The amount of time at the final temperature was 60-120 minutes; cooling down time was about 2 minutes (cooling by submerging the reactors in ice water).

Unreacted sulfur dioxide was removed slowly from the system by venting, and no fiber explosion was used. No separation of pulp and liquor took place. The material was transferred to a Rotary Evaporator flask and evaporated at about 75 °C with stepwise decrease in pressure from atmospheric to about 150 Torr, to provide a final total solids content of about 14-17%. Laboratory type II water was added to bring total solids to about 12%, followed by the second evaporation at the above conditions. Laboratory type II water was added again to bring total solids to about 12%, followed by the third evaporation at the above conditions.

In some experiments, the material was heat treated at various conditions (total solids ^{~}11-14%) using minireactors submerged in oil baths.

In all the experiments, the material was transferred to a shake flask and diluted with laboratory type II water. The pH of the mixture was adjusted by adding a 30% calcium hydroxide slurry to achieve a pH of 4, followed by the pH adjustment with a 15% ammonium hydroxide solution to achieve a pH of 5.4-5.5. Other alkali or alkaline earth metal hydroxides (for example, solutions of KOH and NaOH or slurries of Mg(OH)₂) or oxides (for example, slurries of MgO and CaO) can be used (one or more than one) instead of calcium hydroxide or ammonium hydroxide to achieve required pH levels, as would be readily recognized by one of skill in the art. The material was then subjected to enzymatic hydrolysis for 96 hours. A commercially available enzyme cocktail containing cellulases, hemicellulases and β-glucosidases was used. Total solids content during enzymatic hydrolysis was about 9-10%, the pH was maintained at 4.8-5.5 using 15% ammonium hydroxide solution, and the temperature was maintained at about 53 °C. The shaking rate was 200 RPM.

**Tables 3-6** display the surprisingly high sugar yields at low enzyme charges of 0.8 and 1.6 FPU/o.d. g biomass, and the surprisingly low degradation products. Overall glucose yields of 84-97% (based on the available glucan in the biomass) were obtained despite the presence of high amounts of water-insoluble lignin fractions that are generally inhibitory to enzyme hydrolysis. The overall hemicellulose sugar yield reached 76-95% (based on the available hemicelluloses in the biomass). The overall monosugar yield achieved for corn stover was 81-85% and 83-93% (based on the available polysaccharides in the biomass), at 0.8 and 1.6 FPU/o.d. g biomass enzyme dosage, respectively.

**Table 3. Sugar yields and degradation products in Example 2 (5% sulfur dioxide, 47.5% ethanol).**

| Experiment | No. 2.1 | No. 2.2 | No. 2.3 | No. 2.4 |
|---|---|---|---|---|
| Moisture-adjusted pretreatment liquor composition, by weight | 5% sulfur dioxide, 47.5% ethanol and 47.5% water | | | |
| Pretreatment conditions | Temperature 150°C, duration 60 min | | | |
| Heat Treatment | 4 hr @ 99°C | 4 hr @ 99°C | 4 h @ 120°C | 4 h @ 120°C |
| Enzyme charge, FPU per o.d. g biomass | 0.8 | 1.6 | 0.8 | 1.6 |
| Monosaccharide yield, % on available in biomass | | | | |
| Glucose | 84 | 93 | 84 | 97 |
| Hemicellulose Sugars | 78 | 80 | 86 | 86 |
| Total | 81 | 88 | 85 | 93 |
| Monosaccharide yield, kg per BD tonne biomass | | | | |
| Total | 504 | 548 | 529 | 579 |

The composition was analyzed to measure the content of sugar degradation products, which were found to account for less than about 8 kg per BD tonne of the original biomass (see Table 4), which is unexpectedly low based on the relatively high pretreatment temperature (150 °C).

**Table 4. Sugar yields and degradation products in Example 2 (5% sulfur dioxide, 24% ethanol).**

| Experiment | No. 2.5 | No. 2.6 | No. 2.7 | No. 2.8 |
|---|---|---|---|---|
| Moisture-adjusted pretreatment liquor composition, by weight | 5% sulfur dioxide, 24% ethanol and 71% water | | | |
| Pretreatment conditions | Temperature 150°C, duration 60 min | | | |
| Heat Treatment | None | 4 hr @ 99°C | 8 hr @ 99°C | 1 hr @ 120°C |
| Enzyme charge, FPU per o.d. g biomass | 1.6 | 1.6 | 1.6 | 1.6 |
| Monosaccharide yield, % on available in biomass | | | | |
| Glucose | 93 | 94 | 92 | 94 |
| Hemicellulose Sugars | 86 | 89 | 95 | 90 |
| Total | 90 | 92 | 93 | 93 |
| Monosaccharide yield, kg per BD tonne biomass | | | | |
| Total | 560 | 573 | 579 | 579 |
| Sugar degradation products, kg per BD tonne biomass | | | | |
| Furfural | 0 | NA | 0.5 | 0.1 |
| HMF | 4.4 | NA | 4.6 | 4.6 |
| Levulinic acid | 2 | 2.5 | 2.5 | 3.2 |
| Other compounds, kg per BD tonne biomass | | | | |
| Acetic acid | 12 | 12.9 | 13.5 | 12.4 |

**Table 5. Sugar yields and degradation products in Example 2 (3% sulfur dioxide, 24% ethanol).**

| Experiment | No. 2.9 | No. 2.10 | No. 2.11 |
|---|---|---|---|
| Moisture-adjusted pretreatment liquor composition, by weight | 3% sulfur dioxide, 24% ethanol and 73% water | | |
| Pretreatment conditions | Temperature 150°C, duration 60 min | | |
| Heat Treatment | None | 1 hr @ 99°C | 1 hr @ 120°C |
| Enzyme charge, FPU per o.d. g biomass | 1.6 | 1.6 | 1.6 |
| Monosaccharide yield, % on available in biomass | | | |
| Glucose | 91 | 87 | 94 |
| Hemicellulose Sugars | 89 | 78 | 93 |
| Total | 91 | 83 | 93 |
| Monosaccharide yield, kg per BD tonne biomass | | | |
| Total | 566 | 517 | 579 |
| Sugar degradation products, kg per BD tonne biomass | | | |
| Furfural | 0 | 0 | 0.1 |
| HMF | 3.8 | 4.6 | 4.9 |
| Levulinic acid | 0 | 2.2 | 0 |
| Other compounds, kg per BD tonne biomass | | | |
| Acetic acid | 10.1 | 14.5 | 14.9 |

**Table 6. Sugar yields and degradation products in Example 2 (7% sulfur dioxide, 23% ethanol).**

| Experiment | No. 2.12 | No. 2.13 | No. 2.14 | No. 2.15 |
|---|---|---|---|---|
| Moisture-adjusted pretreatment liquor composition, by weight | 7% sulfur dioxide, 23% ethanol and 70% water | | | |
| Pretreatment conditions | Temperature 140°C, duration 120 min | | | |
| Heat Treatment | None | 4 hr @ 99°C | 1 hr @ 99°C | 1 hr @ 120°C |
| Enzyme charge, FPU per o.d. g biomass | 1.6 | 1.6 | 1.6 | 1.6 |
| Monosaccharide yield, % on available in biomass | | | | |
| Glucose | 90 | 91 | 89 | 92 |
| Hemicellulose Sugars | 80 | 89 | 76 | 95 |
| Total | 86 | 90 | 85 | 92 |
| Monosaccharide yield, kg per BD tonne biomass | | | | |
| Total | 535 | 560 | 529 | 573 |
| Sugar degradation products, kg per BD tonne biomass | | | | |
| Furfural | 0 | 0 | 0 | 0.2 |
| HMF | 2.7 | 4.4 | 3.3 | 3.7 |
| Levulinic acid | 0 | 4.1 | 3.5 | 2.9 |
| Other compounds, kg per BD tonne biomass | | | | |
| Acetic acid | 11.4 | 13.8 | 14.2 | 13.7 |

Based on experimental data and literature data, employing other softwoods, hardwoods, or biomass such as corn stover, will provide similar or higher yields of monosaccharides.

### Example 3. Conventional hot water (hydrothermal) pretreatment and enzyme hydrolysis of corn stover (Comparative example)

For comparison, the Corn Stover batch used in Example 2 was subjected to conventional hot water (hydrothermal) pretreatment followed by enzyme hydrolysis. Laboratory type II water was charged at about 5.5 parts water per 1 part of dry biomass (i.e., at liquor-to-solid ratio of 5.5 kg/kg). The biomass and water were combined, and the pretreatment was carried out at a final temperature of 150-180 °C, which final temperature was achieved about 10 minutes after initiation of the pretreatment. The amount of time at the final temperature was 20-180 minutes; cooling down time was about 2 minutes (cooling by submerging the reactors in ice water).

No separation of pulp and liquor took place. The material was transferred to a shake flask and diluted with laboratory type II water. The pH of the mixture was adjusted by adding a 30% calcium hydroxide slurry to achieve a pH of 4, followed by the pH adjustment with a 15% ammonium hydroxide solution to achieve a pH of 5.4-5.5. The material was then subjected to enzymatic hydrolysis for 96 hours. A commercially available enzyme cocktail containing cellulases, hemicellulases and β-glucosidases was used. Total solids content during enzymatic hydrolysis was about 9-10%, the pH was maintained at 4.8-5.5 using 15% ammonium hydroxide solution, and the temperature was maintained at about 53 °C. The shaking rate was 200 RPM.

The results of the conventional hot water (hydrothermal) pretreatment of corn stover are given in **Table 7.** The monosaccharide yields were quite similar at the four temperatures that were studied. There was little or no correlation between increasing the Pretreatment duration and achieving higher monosaccharaide yields. At enzyme charges of 1.7 and 3.3 FPU per o.d. g biomass, the monosaccharide yields were within the range of 46-68%, which is rather low.

**Table 7. Sugar yields in comparative Example 3 (conventional hot water pretreatment)**

| | | | | | | | | | | | | |
|---|---|---|---|---|---|---|---|---|---|---|---|---|
| Pretreatment temperature, °C | 150 | | | 160 | | | 170 | | | 180 | | |
| Pretreatment duration, min | 120, 180 | | | 60, 90, 120, 150, 180 | | | 40, 60, 80, 120 | | | 20, 30, 40, 60 | | |
| Enzyme charge, FPU per o.d. g biomass | 1.7 | 3.3 | 6.6 | 1.7 | 3.3 | 6.6 | 1.7 | 3.3 | 6.6 | 1.7 | 3.3 | 6.6 |
| Total monosaccharide yield, % on available in biomass | - | 57-58 | 60-72 | 49-54 | 58-67 | 69-72 | 52-62 | 56-65 | 70-77 | 46-57 | 60-68 | 61-73 |

Figure 6 shows the comparison between the conventional hot water (hydrothermal) pretreatment of corn stover (this example; Example 3) and the method described in Example 2. It was observed that the method described in Example 2 reached 85% monosugar yield at 0.8 FPU per o.d. g corn stover (trial No. 2.3) and over 90-93% at 1.6 FPU per o.d. g corn stover (trial Nos. 2.4 to 2.9, 2.11, 2.13, and 2.15) compared to about 46-62% monosugar yield at 1.7 FPU per o.d. g corn stover and about 56-68% monosugar yield at 3.3 FPU per o.d. g corn stover, for the hot water process. Thus, the method described in Example 2 allows achieving monosugar yields that are at least 50% higher than those that can be achieved in conventional hot water process, at a reasonable enzyme charge of 1.6-1.7 FPU per o.d. g biomass.

The processes of Example 2 allow for further decreasing the enzyme charge to 0.8 FPU per o.d. g biomass with very high monosaccharide yield (85%); wherein the yield is higher than can be achieved even at a very high charge of 6.6 FPU per o.d. g biomass for the hot water process (60-77% yield).

### Example 4. Fermentation of the enzyme hydrolyzates obtained in Example 2

The enzyme hydrolysates obtained in Example 2 were fermented to ethanol, in shake flasks (200 mL volume, shaking speed 200 RPM) at pH = 5.0-5.5, in duplicate, by a commercially available genetically modified (GMO) yeast strain capable of utilizing glucose, mannose, galactose, xylose and arabinose. Suitable examples of yeast cells and fermentation techniques are described in US Patent Publication No. 2019/0106464 (Oeser et al.). The fermentation process can optionally employ non-GMO yeast or bacteria strains, GMO yeast or bacteria strains, or a combination of non-GMO and GMO strains. The fermentation was carried out at 32 °C. The fermentation took less than 24 hours, at which time practically all sugars were consumed, and the ethanol yield was 83-87% of theoretical, i.e., 0.42-0.44 g ethanol/g sugar.

Based on experimental data and literature data, conducting the fermentation process on a larger scale than in this example can result in an increase of the fermentation yields by about 3%, up to about 10%. For example, the use of a bioreactor that has a working volume of 1-50 liters can provide an additional 3-8% yield, whereas an industrial fermenter having a working volume of at least 100 cubic meters can provide an additional 5-10% yield, compared to the 0.2-liter scale used for this example.

All publications, patents, and patent documents cited herein are incorporated by reference as though individually incorporated by reference. No limitations inconsistent with this disclosure are to be understood therefrom. The invention has been described with reference to various specific and preferred embodiments and techniques. However, many variations and modifications may be made while remaining within the spirit and scope of the invention.

While specific embodiments have been described above with reference to the disclosed embodiments and examples, such embodiments are only illustrative and do not limit the scope of the invention. Changes and modifications can be made in accordance with ordinary skill in the art without departing from the invention in its broader aspects as defined in the following claims.

## Claims

1. A process for the production of one or more fermentation products and lignin comprising:
(a) contacting lignocellulosic biomass or steamed lignocellulosic biomass with a pretreatment liquor at an elevated temperature under pressure;
wherein the pretreatment liquor comprises sulfur dioxide, volatile alcohol, and water;
thereby releasing and/or dissolving lignin and hemicelluloses from the lignocellulosic biomass into the pretreatment liquor, resulting in a mixture comprising lignin and hemicelluloses dissolved in the pretreatment liquor and a solid fraction comprising mainly cellulose, which mixture is referred to as the pretreated material;
(b1) recovering sulfur dioxide and alcohol, thereby precipitating lignin and producing a first mixture comprising cellulose, lignin, and monosaccharides;
(b2) optionally retaining the first mixture at a desired temperature and at a desired pressure and mixing rate or recirculating rate, whereby the chemically bound volatile alcohol is released and optionally recovered, providing a second mixture comprising cellulose, lignin, and monosaccharides;
(c) adjusting the pH of the first or second mixture to an appropriate level for enzymatic hydrolysis;
(d) contacting the pH-adjusted first or second mixture with a cellulase, a glucosidase, a hemicellulase, or a combination thereof, to provide a third mixture comprising glucose derived from cellulose and monosaccharides derived from hemicelluloses as well as lignin; and
(e) subjecting the third mixture to fermentation;
to produce the one or more fermentation products.

2. The process of claim 1 wherein step (d) further comprises removing one or more of lignin, alkoxylated lignin, and lignosulfonate, from the third mixture.

3. The process of claim 1 or 2 wherein the volatile alcohol of the step (a) pretreatment liquor comprises methanol, ethanol, 1-propanol, 2-propanol, 2-methyl-2-propanol, or a mixture thereof; and optionally, in addition to the volatile alcohol, the pretreatment liquor comprises one or more volatile hydrocarbons, wherein the volatile hydrocarbons are selected from alkanes such as pentane, hexane, cyclopentane, and cyclohexane; ethers such as diethyl ether and MTBE; and mixtures thereof.

4. The process of any one of claims 1-3 wherein in step (b1) and/or step (b2):
sulfur dioxide chemically or loosely bound to sugars, to lignin, or to other compounds, is also released and optionally recovered; and/or
oligosaccharides are hydrolyzed, thereby liberating monosaccharides; and/or
lignin is dealkoxylated, sugars are dealkoxylated, or both lignin and sugars are dealkoxylated.

5. The process of any one of claims 1-4 wherein recovering sulfur dioxide in step (b1) or in steps (b1) and (b2) comprises:
(i) releasing gaseous vapors from the pretreated material at elevated temperature and/or at a lower pressure, wherein the gaseous vapors released from the pretreated material comprise sulfur dioxide and one or more of water vapor and alcohol vapor; wherein the gaseous vapors comprising sulfur dioxide are optionally purified by removing traces of water vapor and organic volatiles;
(ii) condensing at least a portion of the gaseous vapors to provide sulfur dioxide gas and a liquid containing at least one of water and alcohol;
(iii) liquefying sulfur dioxide gas by pressure change and/or temperature reduction of the sulfur dioxide gas, thereby providing liquid sulfur dioxide; and optionally storing the liquid sulfur dioxide; and
(iv) introducing the liquid sulfur dioxide to the container of step (a) or another pretreatment vessel, optionally by pumping.

6. The process of claim 5 wherein ethanol is used as the volatile alcohol and is obtained in step (ii) of claim 5, condensed and recycled back to pretreatment and/or at least partly combined with fermentation broth and recovered together with the ethanol produced in fermentation.

7. The process of any one of claims 1-6 wherein the step (a) pretreatment is performed in a continuous mode or in a batch mode.

8. The process of any one of claims 1-7 wherein the step (b1), step (b2) or both, is/are accomplished using one or more of blow tank(s), stripping column(s), distillation column(s), and fractionation column(s), and/or wherein the step (b1), step (b2) or both, is/are accomplished in one or more of containers that are tanks equipped with a heated recirculation line and a distillation column, fractionating column, or a stripper, wherein the column is constructed with trays, packing, or a combination thereof, and transferring pretreated material to said tank or tanks is accomplished by pressure difference (blow) or pumping.

9. The process of any one of claims 1-8 wherein there is no separation of the cellulose from the remainder of the first or second mixture between step (b1) and step (d), such that cellulose and precipitated lignin are both present in the first or second mixture of step (d).

10. The process of any one of claims 1-9 wherein step (d) is performed with an enzyme charge of less than 5 FPU, less than 4 FPU, less than 3.5 FPU, less than 3 FPU, less than 2.5 FPU, less than 2 FPU or 0.2 to 5 FPU, 0.5 to 3.5 FPU, 0.5 to 3.3 FPU, 0.5 to 2.5 FPU, 0.5 to 2 FPU, or 0.8 to 1.6 FPU per o.d. g biomass.

11. The process of any one of claims 1-9 wherein the step (d) enzymatic hydrolysis is performed in a continuous mode or in a batch mode and/or wherein in the step (d) enzymatic hydrolysis, the pretreated substrate is added at once, in batches, or stepwise, and the enzyme is added at once, in batches, or stepwise.

12. The process of any one of claims 1-11 wherein the step (d) enzymatic hydrolysis is performed at a total solids content of higher than 9%.

13. The process of any one of claims 1-12 wherein lignin is partly or wholly separated after enzymatic hydrolysis (d), or after fermentation (e); or wherein alkoxylated lignin is partially or wholly dealkoxylated in steps (b1) and/or (b2), and the resulting lignin material is partly or wholly separated after enzymatic hydrolysis (d), or after fermentation (e).

14. The process of any one of claims 1-13 wherein the fermentation product is ethanol.

15. The process of any one of claims 1-14 wherein prior to step (a), the lignocellulosic biomass is contacted with steam, wherein the lignocellulosic biomass absorbs water from the steam, thereby removing air from the lignocellulosic biomass.
